(19) Europäisches Patentamt
European Patent Office
Office européen des brevets

(11) Veröffentlichungsnummer: **0 121 838**
**B1**

(12) # EUROPÄISCHE PATENTSCHRIFT

(45) Veröffentlichungstag der Patentschrift: **26.02.86**

(51) Int. Cl.⁴: **C 07 C 45/59**, C 07 C 49/203

(21) Anmeldenummer: **84103148.7**

(22) Anmeldetag: **22.03.84**

(54) **Verfahren zur Herstellung von in 1,4-Stellung substituierten 2-Buten-1,4-dionen.**

(30) Priorität: **29.03.83 DE 3311320**

(43) Veröffentlichungstag der Anmeldung: **17.10.84 Patentblatt 84/42**

(45) Bekanntmachung des Hinweises auf die Patenterteilung: **26.02.86 Patentblatt 86/9**

(84) Benannte Vertragsstaaten: **BE CH DE FR GB IT LI**

(56) Entgegenhaltungen:
**EP - A - 0 032 290**
**DE - A - 3 136 987**
**DE - C - 809 806**

**CHEMICAL ABSTRACTS, Band 54, 1960 Columbus, Ohio, USA SHINICHI KAWAI et al., "Oxidative ring cleavage of 2,5-dimethylfuran" Spalten 24359i-25360b**
**BULLETIN DE LA SOCIETE CHIMIQUE DE FRANCE, 1957 J. LEVISALLES, "Pyridazines. II. La préparation des pyridazines à partir de dérivés du furanne" Seiten 997-1003**

(73) Patentinhaber: **BASF Aktiengesellschaft, Carl-Bosch-Strasse 38, D-6700 Ludwigshafen (DE)**

(72) Erfinder: **Fischer, Rolf, Dr., Bergstrasse 98, D-6900 Heidelberg (DE)**
Erfinder: **Weitz, Hans-Martin, Dr., Auf dem Koeppel 40, D-6702 Bad Duerkheim (DE)**
Erfinder: **Siegel, Hardo, Dr., Hans-Purrmann-Allee 25, D-6720 Speyer (DE)**

**EP 0 121 838 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die Erfindung betrifft ein Verfahren zur Herstellung von in 1,4-Stellung substituierten 2-Buten-1,4-dionen durch Oxidation von 2,5-disubstituier-ten Furanen mit aliphatischen Percarbonsäuren.

Für die Herstellung von 1,4-Dialkyl-2-buten-1,4-dionen aus 2,5-Dialkyl-furanen sind schon mehrere Synthesewege bekannt. So läßt sich 3-Hexen-2,5-dion durch Oxidation von 2,5-Dimethylfuran mit einer Komplexverbindung aus Chromylchlorid und Pyridin (DE-OS 31 36 987) oder durch Hydrolyse von aus 2,5-Dimethylfuran zugänglichem 2,5-Dimethoxy-2,5-dimethyl-2,5-dihydro-furan (Bull, Soc. Chim. France, 1957, Seiten 997-1003) herstellen. 2,2,7,7-Tetramethyl-4-octen-3,6-dion ist durch Umsetzung von 2,5-Di-tert.-butylfuran mit anorganischen Hypochloriten zugänglich (EP 32 290).

Diese Verfahren haben den Nachteil, daß mehrere Reaktionsschritte notwendig sind, toxische Oxidationsmittel verwendet werden und/oder Neutralsalze bei der Umsetzung anfallen, die entsorgt werden müssen.

Es wurde nun gefunden, daß man in 1,4-Stellung substituierte 2-Buten-1,4-dione der Formel

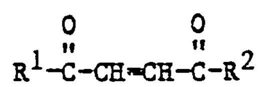

I,

in der $R^1$ und $R^2$ aliphatische Kohlenwasserstoffreste mit 1 bis 17 Kohlenstoffatomen bedeuten, durch Oxidation von 2,5-disubstituierten Furanen der Formel

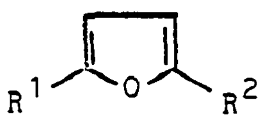

II,

in der $R^1$ und $R^2$ die oben genannte Bedeutung haben, erheblich vorteilhafter herstellen kann, wenn män die Oxidation mit aliphatischen Percarbonsäuren und in Gegenwart von aliphatischen Carbonsäuren vornimmt.

Die Reaktion läßt sich für den Fall der Oxidation von 2,5-Dimethylfuran mit Peressigsäure in Essigsäure zum 3-Hexen-2,5-dion durch die folgenden Formeln veranschaulichen:

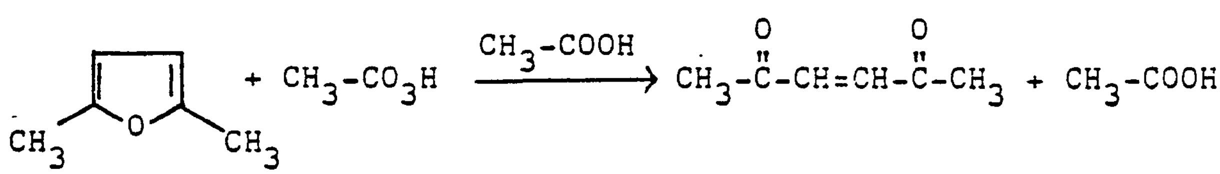


Das Verfahrensergebnis war nicht vorherzusehen, denn durch Umsetzung von Furan mit Peressigsäure oder Perbenzoesäure erhält man Maleindialdehyd in schlechter Ausbeute (Acta Chem. Scandinavia 1, Seiten 419-420 (1947). Oxidiert man Furan mit p-Nitroperbenzoesäure in Ether, so isoliert man 3-Formylacrylsäure in nur 12 %iger Ausbeute (Comptes rendues Acad. Sciences Paris, 258, Seiten 4094-4096 (1964). Im Gegensatz hierzu erhält man bei der Umsetzung von 2-Methylfuran mit Peressigsäure in Eisessig geringe Mengen 3-Hydroxy-5-methyl-2-(2,3 H)-furanon. 90 % des eingesetzten 2-Methylfurans gehen dabei in harzartige Produkte über (Rec. trac. chim. 50, Seiten 1029-1034 (1931). Ebenso wenig ließen die Angaben über die Umsetzung von Alkylfuranen mit Wasserstoffperoxid Vorausaagen über das erfindungsgemäße Verfahren zu. So entsteht nämlich durch Behandlung von 2-Methylfuran mit Wasserstoffperoxid in Gegenwart von Natriumstannat 5-Methyl-2(5H)-furanon (SU-PS 833 965), und bei der Umsetzung von 2,5-Dimethylfuran mit Wasserstoffperoxid werden je nach Reaktionsbedingungen drei verschiedene cyclische Bishydroperoxide erhalten (Chem. Berichte 96, Seiten 2712-2722 (1963).

In den Formeln I und II bedeuten die Substituenten $R^1$ und $R^2$ aliphatische Kohlenwasserstoffreste, wie Alkyl- oder Cycloalkylreste mit 1 bis 17 C-Atomen, wie Methyl-, Ethyl-, n-Propyl-, i-Propyl-, n-Butyl-, i-Butyl-, sec.-

Butyl-, tert.-Butyl-, Hexyl-, Octyl-, Palmityl-, Stearyl-, Cyclopentyl-, Cycloheptyl- oder Cyclohexylreste.

Die Ausgangsstoffe der Formel II lassen sich z.B. durch intramolekulare Kondensation von 1,4-Dialkyl-2-butan-1,4-dionen in Gegenwart von Mineralsäuren (DE-OS 20 13 261) oder von sauren Ionenaustauschern (J. Org. Chem. 45, Seiten 5048-5052) oder durch katalytische Hydrierung von 5-Alkylfur-furalen (SU-PS 844 617) herstellen. Beispielsweise seien die folgenden Ausgangsstoffe der Formel II genannt: 2,5-Dimethylfuran, 2-Methyl-5-ethyl-furan, 2,5-Di-tert.-butylfuran, 2,5-Dicyclohexylfuran, 2,5-Dipalmityl-furan, 2-Methyl-5-cyclopentylfuran.

Als Oxidationsmittel verwendet man aliphatische Percarbonsäuren, wie Percarbonsäuren der Formel $R^3$-$CO_3H$ (III) und als aliphatische Carbonsäuren z.B. solche der Formel $R^4$-COOH (IV). Die Reste $R^3$ und $R^4$ bedeuten Alkylreste oder Cycloalkylreste mit 1 bis 17 C-Atomen, vorzugsweise Alkylreste mit 1 bis 5 C-Atomen.

Als Percarbonsäuren der Formel III verwendet man beispielsweise Perameisensäure, Peressigsäure, Perpropionsäure, Per-n-Buttersäure oder Per-i-Buttersäure. Die Persäure braucht nicht in wasserfreier Form verwendet zu werden. Sie kann beispielsweise auch als wasserhaltige ""Gleichgewichts-Persäure'' eingesetzt werden. Zur Herstellung der ""Gleichgewichtspersäure'' wird eine aliphatische Carbonsäure, wie Essigsäure in Gegenwart von konzentrierter Schwefelsäure sls Katalysator mit 50 %igem Wasserstoffperoxid umgesetzt. Vor Verwendung der Persäure wird die Schwefelsäure neutralisiert.

Als Carbonsäuren der Formel IV kommen z.B. Ameisensäure, Essigsäure, Propionsäure, Buttersäure, Valeriansäure, Caprinsäure, Laurinsäure, Ölsäure, Palmitinsäure und Cyclohexancarbonsäure in Betracht. Essigsäure ist aus wirtschaftlichen Gründen besonders bevorzugt. Die Carbonsäure wird im allgemeinen im Überschuß, z.B. in der 1- bis 80-molaren Menge, bezogen auf die eingesetzte Furanverbindung II, zugesetzt.

Die Umsetzung der Furane mit den Percarbonsäuren in Gegenwart der Carbonsäuren wird z.B. so durchgeführt, daß man pro Mol Furanverbindung 1 bis 80 Mole, insbesondere 1,5 bis 60 Mole der Carbonsäure und 0,5 bis 2 Mole, insbesondere 1 bis 1,5 Mole der Percarbonsäure anwendet. Man arbeitet zweckmäßig bei Temperaturen zwischen 0 und 200°C, insbesondere bei 20 bis 120°C. Gewöhnlich arbeitet man entweder in einem Überschuß der Carbonsäure als Lösungsmittel oder zusätzlich in Gegenwart von unter den Reaktionsbedingungen inerten Lösungsmitteln. Als Lösungsmittel dieser Art kommen z.B. Carbonsäureester, wie Essigsäuremethylester, chlorierte Kohlenwasserstoffe, wie Methylenchlorid, Chloroform, Tetrachlorkohlenstoff oder 1,2-Dichlorethan, Kohlenwasserstoffe, wie Alkane, Benzol und alkylierte Benzole, Ether, wie Diethylether, Tetrahydrofuran oder Dioxan in Betracht. Pro Mol Ausgangsverbindung II verwendet man zweckmäßigerweise 0,1 bis 80 Mol, insbesondere 2 bis 60 Mol des unter den Reaktionsbedingungen inerten Lösungsmittels.

Das neue Verfahren kann chargenweise oder kontinuierlich, drucklos oder unter Druck durchgeführt werden. Unumgesetzte Furane der Formel II können gegebenenfalls nach der Umsetzung destillativ von den entstandenen 2-Buten-1,4-dionen der Formel I abgetrennt und erneut für die erfindungs-gemäße Umsetzung verwendet werden.

Die Umsetzung nimmt man bei diskontinierlicher Arbeitsweise z.B. wie folgt vor: Zu einer Lösung der Furanverbindung in der Carbonsäure, die gegebenenfalls noch das Lösungsmittel enthält, wird bei der Reaktionstemperatur und dem Reaktionsdruck die Percarbonsäure zugegeben. Nach beendeter Zugabe wird gegebenenfalls noch nachgerührt. Nach Prüfung auf unumgesetzte Perverbindungen und dem Abdestillieren des Lösungsmittels und/oder der Carbonsäure wird fraktioniert destilliert oder umkristallisiert. Bei der Destillation werden gegebenenfalls nicht umgesetzte Ausgangsverbindungen von den gewünschten 2-Buten-1,4-dionen abgetrennt.

Die in 1,4-Stellung substituierten 2-Buten-1,4-dione werden als Gemische der (E)- und (Z)-Isomeren erhalten. Da sich die (E)-Isomeren durch Bestrahlung in die (Z)-Isomeren (DE-OS 31 36 987), die (Z)-Isomeren, z.B. durch Erhitzen mit Iod (Journ. Heterocyclic Chemistry, Seite 528 (1972) in die (E)-Isomeren überführen lassen, sind die jeweils gewünschten Isomeren, ausgehend von den erhaltenen Isomerengemischen, zugänglich.

Die nach dem erfindungsgemäßen Verfahren herstellbaren 1,4-Dialkyl-2-buten-1,4-dione II stellen wertvolle Zwischenprodukte dar. Sie sind beispielsweise für die Herstellung von Pyridazinen (Journal Heterocyclic Chemistry, Seiten 523-529 (1972) geeignet. 1,4-Dialkyl-2-buten-1,4-dione II lassen sich auch für die Herstellung der sogenannten Retrolone verwenden, die für die Synthese der insektiziden Pyrethrine eingesetzt werden (DE-OS 28 19 879 und 31 36 987).

Nach dem Verfahren der Erfindung erhält man die gewünschten in 1,4-Stel-lung substituierten 2-Buten-1,4-dione glatt und auf einfachem Wege. Das Verfahren eignet sich wegen der Verwendung gut zugänglicher Ausgangsstoffe und wegen der Vermeidung toxischer Oxidationsmittel hervorragend für die Durchführung in technischem Maßstab. Der Anfall von Salzen und damit verbundene Entsorgungsprobleme werden ebenfalls vermieden.

**Beispiel 1**

Zu einer Lösung von 4,3 g 2,5-Dimethylfuran in 5 g Eisessig wurden bei 40°C unter Rühren und Kühlen innerhalb von 20 Minuten 34,2 g wasserfreie Peressigsäure (12 Gew.%) in Eisessig (hergestellt nach Houben-Weyl, 4. Auflage, Methoden der organischen Chemie, Band 8, Seite 41) getropft. Man rührte 30 Minuten bei 40-C nach. Nach dieser Zeit waren im Reaktionsgemisch laut iodometrischer Titration nur noch 1,5 Mo1.% Peressigsäure

(bezogen auf eingesetzte Peressigsäure) enthalten.

Nach Abziehen des Eisessigs am Rotationsverdampfer erhielt man durch Kugelrohrdestillation (60 bis 100° C/0,7 mbar) 3,4 g flüssiges 3-Hexen-2,5-dion (68 %, bezogen auf eingesetztes 2,5-Dimethylfuran),

$$= n_D^{20} = 1,4560.$$

$^1$H-NMR-Spektrum ($CDCl_3$):
(Z)-3-Hexen-2,5-dion: $\delta$ = 2,20 (s, 6H), 6,30 (s, 1H)
(E)-3-Hexen-2,5-dion: $\delta$ = 2,35 (s, 6H), 6,75 (s, 1H)

Das Verhältnis der Methylprotonen zu den olefinischen Protonen zeigte, daß ein Gemisch, bestehend aus 75 % (Z)- und 25 % (E)-3-Hexen-2,5-dion vorlag.

**Beispiel 2**

Ein Gemisch aus 500 g Eisessig, 52 g Wasserstoffperoxid (50 %) und 2,5 g konzentrierter Schwefelsäure wurde 48 Stunden bei Raumtemperatur gerührt. 260 g der so hergestellten Gleichgewichtsperessigsäure wurden zur Neutralisation der Schwefelsäure kurz vor der Umsetzung mit 2,5-Dimethylfuran mit 2,8 g Kaliumacetat versetzt.

29 g 2,5-Dimethylfuran, gelöst in 30 g Eisessig, wurden unter Rühren und Kühlung bei 40° C innerhalb von 35 Minuten mit 260 g der Gleichgewichtsperessigsäure (10,5 Gew.%) versetzt. 20 Minuten lang wurde bei 40° C nachgerührt. Der Restperessigsäuregehalt, der wie im Beispiel 1 ermittelt wurde, betrug 1,4 Mo1.% (bezogen auf eingesetzte Peressigsäure). Das $^1$H-NMR-Spektrum des Reaktionsgemisches ergab keine Hinweise für das Auftreten von cyclischen Peroxiden, wie sie bei der Umsetzung von 2,5-Dimethylfuran mit 80 bis 90 %igem Wasserstoffperoxid beobachtet wurden (Chem. Ber. 96, Seiten 2712-2722 (1963).

Nach Abziehen der Essigsäure am Rotationsverdampfer und Filtration des ausgefallenen Kaliumsulfats wurden durch Destillation 21,3 g 3-Hexen-2,5-dion vom Siedepunkt 95 bis 100° C/17 mbar (63 %, bezogen auf eingesetztes 2,5-Dimethylfuran) erhalten. Das $^1$H-NMR-Spektrum zeigte, daß das Isomerengemisch zu 30 % aus (Z)-, zu 70 % aus (E)-3-Hexen-2,5-dion bestand.

**Patentansprüche**

1. Verfahren zur Herstellung von in 1,4-Stellung substituierten 2-Buten-1,4-dionen der Formel

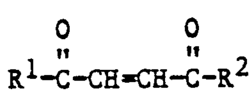 I,

in der R¹ und R² aliphatische Kohlenwasserstoffreste mit 1 bis 17 Kohlenstoffatomen bedeuten, durch Oxidation von 2,5-disubstituierten Furanen der Formel

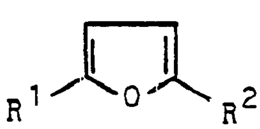 II,

in der R¹ und R² die oben genannte Bedeutung haben, dadurch gekennzeichnet, daß man die Oxidation mit aliphatischen Percarbonsäuren und in Gegenwart von aliphatischen Carbonsäuren bei Temperaturen von 0 bis 200° C vornimmt.

2. Verfahren nach Anspruch 1, dadurch gekennzeichnet, daß man aliphatische Percarbonsäuren der Formel $R^3$-$CO_3H$ und aliphatische Carbonsäuren der Formel $R^4$-COOH, in denen die Reste $R^3$ und $R^4$ Alkyl- oder

Cycloalkylreste mit 1 bis 17 C-Atomen bedeuten, verwendet.

3. Verfahren nach Anspruch 1, dadurch gekennzeichnet, daß man als Percarbonsäure Perameisensäure, Peressigsäure, Perpropionsäure, Per-n-Buttersäure oder Per-i-Buttersäure verwendet.

4. Verfahren nach Anspruch 1, dadurch gekennzeichnet, daß man als Carbonsäure Ameisensäure, Essigsäure, Buttersäure, Propionsäure, Valeriansäure, Laurinsäure, Ölsäure, Palmitinsäure oder Cyclohexancarbonsäure verwendet.

## Claims

1. A process for the preparation of a but-2-ene-1,4-dione substituted in the 1,4-positions, of the formula

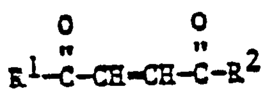

I

where $R^1$ and $R^2$ are each an aliphatic hydrocarbon radical of 1 to 17 carbon atoms, by oxidizing a 2,5-disubstituted furan of the formula

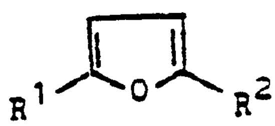

II

where $R^1$ and $R^2$ have the above meaning, wherein the oxidation is carried out using an aliphatic percarboxylic acid and in the presence of an aliphatic carboxylic acid at from 0 to 200°C.

2. A process as claimed in claim 1, wherein an aliphatic percarboxylic acid of the formula $R^3$-$CO_3H$ and an aliphatic carboxylic acid of the formula $R^4$-COOH, where $R^3$ and $R^4$ are each an alkyl or cycloalkyl radical of 1 to 17 carbon atoms, are used.

3. A process as claimed in claim 1, wherein the percarboxylic acid used is performic acid, peracetic acid, perpropionic acid, per-n-butyric acid or per-i-butyric acid.

4. A process as claimed in claim 1, wherein the carboxylic acid used is formic acid, acetic acid, butyric acid, propionic acid, valeric acid, lauric acid, oleic acid, palmitic acid or cyclohexanecarboxylic acid.

## Revendications

1. Procéde pour la préparation de 2-butène-1,4-diones substituées en positions 1 et 4 de formule

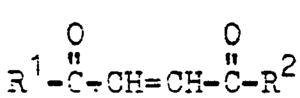

(I)

dans laquelle $R^1$ et $R^2$ représentent des radicaux hydrocarbonés aliphatiques à 1 - 17 atomes de carbone, par oxydation de furannes bisubstituées en positions 2 et 5 de formule

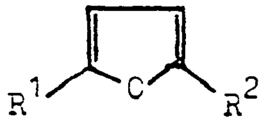

(II)

dans laquelle $R^1$ et $R^2$ ont les significations donées ci-dessus, caractérisé en ce qu'on procèce à l'oxydation avec des acides percarboxyliques aliphatiques et en présence d'acides carboxyliques aliphatiques, à des températures de 0 à 200° C.

2. Procédé selon la revendication 1, caractérisé en ce qu'on utilise des acides percarboxyliques aliphatiques de formule $Rf^3$-$CO_3H$ et des acides carboxyliques aliphatiques de formule $R^4$-COOH, formules dans lesquelles $R^3$ et $R^4$ représentent des radicaux alkyle ou cycloalkyle à 1 - 17 atomes de C.

3. Procédé selon la revendication 1, caractérisé en ce qu'on utilise, en tant qu'acide percarboxylique, l'acide performique, l'acide peracétique, l'acide perpropionique, l'acide per-nbutyrique ou l'acide per-i-butyrique.

4. Procédé selon la revendication 1, caractérisé en ce qu'on utilise, en tant qu'acide carboxylique, l'acide formique, l'acide acétique, l'acide butyrique, l'acide propionique, l'acide valérique, l'acide laurique, l'acide oléique, l'acide palmitique ou l'acide cyclohexane-carboxylique.